(19) Europäisches Patentamt
European Patent Office
Office européen des brevets

(11) **EP 2 412 433 A2**

(12) **EUROPEAN PATENT APPLICATION**

(43) Date of publication:
**01.02.2012 Bulletin 2012/05**

(21) Application number: **11173858.9**

(22) Date of filing: **13.07.2011**

(51) Int Cl.:
**B01J 20/26** *(2006.01)*   **B01J 20/32** *(2006.01)*
**B01J 20/286** *(2006.01)*   **B01J 39/26** *(2006.01)*
**B01J 41/20** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.07.2010 US 368390 P**

(71) Applicant: **Rohm and Haas Company**
**Philadelphia,**
**Pennsylvania 19106-2399 (US)**

(72) Inventors:
• **Deetz, Martin J.**
**North Wales, PA 19454 (US)**
• **Maikner, John J.**
**Old Zionsville, PA 18068 (US)**

(74) Representative: **Buckley, Guy Julian**
**Patent Outsourcing Limited**
**1 King Street**
**Bakewell**
**Derbyshire DE45 1DZ (GB)**

(54) **Grafting method to improve chromatography media performance**

(57) The invention also relates to improvements in the method of grafting polymeric ligands onto substrates used in protein separations, resulting in substrates having improved protein binding capacity, improved purification process operating windows and resin selectivity, and relating to making and using the same.

**EP 2 412 433 A2**

**Description**

The present invention relates to improved methods for grafting polymeric ligands. The invention also relates to improvements in the method of grafting polymeric ligands onto substrates used in protein separations, resulting in substrates having improved protein binding capacity, improved purification process operating windows and resin selectivity, and relating to making and using the same.

Therapeutic proteins produced from living organisms play an increasingly important role in modern healthcare. These proteins provide many advantages over traditional phaz-znaceuticals, including increased specificity and efficacy towards disease targets. Mammalian immune systems use a range of proteins to control and eliminate disease threats. The advent of genetic and protein engineering has allowed the development of many "designed" or recombinant protein therapeutics. These therapeutics can be based on a single protein, chemically modified protein, protein fragment or protein conjugate. One subclass of these therapeutic proteins, monoclonal antibodies (MAbs), has found a wide range of applications in healthcare and diagnostics. Chromatographic separations are extensively utilized in the manufacturing of these biopharmaceuticals. As the industry matures, implementation of novel/advanced technologies and methods to enhance separations will provide biotherapeutic producers the ability to provide these medicines to more patients and at lower cost.

Common chromatography methods used to purify proteins include affinity, bioaffinity, ion exchange, reversed phase, hydrophobic interaction, hydrophilic interaction, size exclusion and mixed mode (combinations of the aforementioned categories) among others. The application and efficiency of each of those types of chromatography procedures relies on the selectivity of surface-surface interactions between the solute molecules and the stationary phase of the chromatography system (chromatography media), each interacting with the mobile liquid phase. A wide variety of stationary phase chromatography support materials are commercially available.

Often the key to a successful separation of product from impurities relies on the correct combination of stationary phase, base matrix and ligand properties (ligand type, ligand density, pore structure, ligand distribution, material composition), and mobile phase or solution properties (buffer type, pH and conductivity). The specific design of the base matrix and ligand results in a chromatography media which can be characterized by several key attributes including protein binding capacity or throughput, selectivity, bed permeability and chemical stability. Purification methods include predominately binding the product (bind and elute), predominately binding the impurities (flow-through) and combinations of the aforementioned (so called weak partitioning and others). It is critical in the design of these technologies to control the chromatography media properties taught above in order to enable and ensure a robust separation leading to purified protein product.

Protein separations can be accomplished on a variety of substrates or base matrices. Common materials for resin or bead structures include polysaccharides (agarose, cellulose), synthetic polymers (polystyrene, polymethacrylate and polyacrylamide) and ceramics such as silica, zirconia and controlled pore glass. These materials adsorb proteins via "diffusive pores" which are typically about 200 Å to 3,000 Å, much smaller than the "convective pores" which are typically about >5$\mu$m.

Membrane and monolith materials are also commonly used for chromatography, particularly flow-through applications. Typical membrane compositions include synthetic polymers such as polyvinylidenefluoride, polyethylene, polyethersulfone, nylon, and polysaccharides such as cellulose.

Monoliths have been developed from polystyrene, polysaccharides, polymethacrylate and many other synthetic polymers. Membrane and monolith chromatography differs from beads in that these materials adsorb proteins in the same "convective pores" which control the membrane and monolith material's permeability. Typical membrane and monolith convective pore sizes range from about 0.6$\mu$m to about 10$\mu$m. Ligand addition to these substrates can be accomplished through a variety of well developed techniques.

The use of ligand "tentacles" or "extenders" to improve protein binding capacity and modify resin selectivity involves placing a ligand on polymer chains coupled to a base matrix such as by grafting, and extend away from the base matrix surface. Ligand extenders typically create greater binding capacity because the extenders increase ligand availability where target molecule binding exceeds that of a monolayer adsorption on the surface.

Two standard methodologies for grafting polymeric ligands have been developed for creating surface extenders on substrates such as those used in chromatography for protein separation and the like: 1) grafting of monomers from a support via a surface radical ("grafting monomers from"), and 2) grafting a preformed polymer to a support via an activating group ("grafting polymers to").

Grafting monomers from materials using radical polymerization reactions is a well developed technology. In general, the reaction can be initiated from a surface material, or from an initiator in solution.

Initiating the radical polymerisation from the surface can be accomplished by generating radicals at the surface via exposure to reactive environments such as radiation, metal oxidation and adsorbed initiating species. However, these "grafting monomers from" approaches require very controlled solution conditions and/or special equipment which makes their implementation complicated and time consuming.

One method for grafting polymers to a support is disclosed in Ruixin Wang, et. al., "Studies on Preparation of PGMA/ Al2O3 and its Effect on Impact Strength of Epoxy Resin" Journal of Applied Polymer Science, Vol. 113, 41-48 (2009). This publication discloses a method of grafting polyglycidyl-methacrylate (PGMA) onto alumina surfaces using peroxide groups as initiators to create modified $Al_2O_3$. The first step in this method includes the chlorination of the hydroxy groups on $Al_2O_3$ by $SOCl_2$. The chloride groups were then treated with tert-butylhydroperoxide to form immobilized peroxide molecules. In a further step the covalently bonded peroxide groups initiated the graft polymerization of GMA on $Al_2O_3$ surfaces. The GMA/$Al_2O_3$ particles were use to impart strength on epoxy resins. Wang does not disclose polymeric substrates and applications involving such.

The addition of polymeric ligands to material surfaces provide improved protein binding capacity and desired potential changes in resin selectivity. However, as protein separations become more demanding, it becomes more critical to develop new technologies and methods in order to create novel polymeric structures. Accordingly, it would be desirable to develop improved protein binding capacity and modify resin selectivity of polymeric substrates used in protein separation.

In response to the above needs for new polymeric substrates, useful for protein separations, having improved protein binding capacity and resin selectivity, a new method for grafting polymeric ligands onto polymeric substrates has been developed.

According to the present invention there is provided a method of preparing an adsorbent material for chromatography comprising:

> (i) providing a polymeric substrate comprising a functional group
> (ii) covalently attaching a free radical ligand to the functional group to form a surface reactive group
> (iii) reacting by free radical the surface reactive group with a vinyl monomer to form a polymeric ligand.

In another aspect of the present invention, there is provided an adsorbent material for chromatography comprising:

> a polymeric ligand immobilized onto a polymeric substrate,
> wherein the polymeric substrate comprises a functional group covalently attached to a free radical ligand to form a surface reactive group
> and further wherein the surface reactive group is reacted by free radical with a vinyl monomer to form a polymeric ligand.

The present invention provides, at least in part, a new method for grafting polymeric ligands onto polymeric substrates, including radical grafting to surface reactive groups that readily generate free radicals.

In the present invention the polymeric substrates are modified by covalently attaching a surface reactive group onto a functional group of the polymeric substrate surface. This attachment is followed by a free radical reaction with a vinyl monomer to form a polymeric ligand. Examples of this embodiment include but are not limited to functionalization of epoxide containing substrates with tert-butylhydroperoxide (tBHP) or peracetic acid and the subsequent functionalization of peroxy modified substrates with (3-acrylamidopropyl)-trimethylammonium chloride (AMPTAC).

All ranges taught herein are to be understood to encompass all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, e.g., 5.5 to 10.

Before describing the present invention in further detail, a number of terms will be defined. Use of these terms does not limit the scope of the invention but only serve to facilitate the description of the invention.

As used herein, the singular forms "a," "an," and "the" include plural referents unless the context clearly dictates otherwise.

" Polymer substrates" or "base matrices" or " substrates" that can be used herein include, but are not limited to, any polymeric material modified with a covalently bound free radical initiator. Suitable structures for polymer substrates include membranes, particles, surfaces, and monoliths. Suitable materials for substrates or base matrices that can be used herein include polysaccharides, synthetic polymer, agarose, cellulose, polymethacrylates, polyacrylates, polyacrylamides, polystyrene, and hybrids or combinations of the aforementioned.

Examples of "functional groups" that can be used herein include, but are not limited to, electrophilic groups capable of reacting with a molecule to form a surface reactive group, such as epoxides, alkyl halides, activated alcohols, activated esters.

The functional groups on the polymeric substrates are functionalized with a free radical ligands to form a functionalized polymer. These functionized polymers are used to covalently bond various polymeric ligands onto the substrate.

The term "free radical ligand." as used herein are any groups capable of reacting with a functional group to form an surface reactive group. Such free radical ligands include but are not limited to peroxides, such as tert-butylhydroperoxide, cumene hydroperoxide; peroxyacetates, such as peracetic acid, chloroperbenzoic acid; persulfates, such as ammonium persulfate, sodium persulfate, potassium peroxodisulfate, azo, among others.

As used herein by "surface reactive group" is meant an immobilized polymerizable group on the polymeric substrate.

In some embodiments a free radical is generated from surface reactive groups either by heating or by redox reaction.

As used herein by "polymeric ligands" is meant a polymer which is covalently immobilized on the polymeric substrate.

Polymeric ligands of the present invention are formed by the free radical polymerization of vinyl monomers onto the polymeric substrate. Examples of vinyl monomers suitable in the present invention include but are not limited to methacrylates, acrylates, methacrylamides and acrylamides and combinations thereof. The monomers include, but are not limited to, acrylic acid, 2-acrylamido-2-methyl-1-propanesulfonic acid, [3-(methacryloylamino) propyl] trimethylammonium chloride, 2-acrylamido-glycolic acid, itaconic acid or ethyl vinyl ketone, glycidyl methacrylate, N,N-dimethylacrylamide, acrylamide, hydroxypropyl methacrylate, N-phenylacrylamide, hydroxyethyl acrylamide, and combinations thereof.

Examples of polymeric ligands that can be used herein include, but are not limited to polymers containing, ion exchange groups, hydrophobic interaction groups, hydrophilic interaction groups, thiophilic interactions groups, metal affinity groups, affinity groups, bioaffinity groups, and mixed mode groups (combinations of the aforementioned). Examples of suitable ligands that can be used herein include, but are not limited to, strong cation exchange groups, such as sulphopropyl, sulfonic acid; strong anion exchange groups, such as trimethylammonium chloride; weak cation exchange groups, such as carboxylic acid; weak anion exchange groups, such as N,N diethylamino or DEAE; hydrophobic interaction groups, such as phenyl, butyl, propyl, hexyl; and affinity groups, such as Protein A, Protein G, and Protein L and unfunctional monomers or intermediary monomers capable of further transformation into another functional group (e.g. glycidyl methacrylate which is transformed into an affinity ligand), and mixtures thereof.

It is further contemplated that during the ligation of the polymeric ligand that additional initiator(s) can be added to the reaction mixture. For example, persulfate, azo and/or peroxide initiator(s) can be added to the reaction mixture

In some embodiments of this chemistry, it may be relevant to add chain transfer reagents during the ligation of the polymeric ligand. Suitable chain transfer agents include, for example, halomethanes, disulfides, thiols (also called mercaptans), and metal complexes. Additional suitable chain transfer agents include various other compounds that have at least one readily abstractable hydrogen atom, and mixtures thereof. Chain transfer agents may be added in one or more additions or continuously, linearly or not, over most or all of the entire reaction period or during limited portions of the reaction period.

Crosslinkers, branching agents and nonfunctional monomers may be attached on the polymeric ligand for the purpose of controlling the morphology or interaction of the polymeric ligands. However, these crosslinkers or branching agents on the polymeric ligand are present at low levels, suitable from <5%, more preferably <1%. Suitable crosslinkers or branching agents include but are not limited to monomers, such as ethylene glycol dimethacrylate, divinyl benzene, trimethylpropyl trimethacrylate and methylene bisacrylamide or multifunctional chain transfer agents

## TEST METHODS

## MEASUREMENT OF BSA CAPACITY

Preparation of Solution 1 (50mM Tris/HCl, pH 8.8)

12.1 g of Tris(hydroxymethyl) aminomethane (Fisher Scientific) was added to a 2 L volumetric flask. Then 0.01N HCl solution (Fisher Scientific) was applied to fill the flask to 2 liter mark. The contents were shaken after the volumetric flask was capped. The solution rested for 5 min and the volume of solution was rechecked. Additional HCl was added to adjust the volume to 2 L mark. The pH was measured at $8.8 \pm 0.05$. The solution was labeled and refrigerated at 4C.

Preparation of Solution 2: 2 mg/ml BSA (albumin from bovine serum, Sigma-Aldrich) solution

0.806 g of BSA was weighed in a glass jar and added into 403 g of Solution 1. The contents were mixed gently to dissolve. The solution sat for 0.5 hour to ensure full BSA dissolution.

BSA capacity uptake procedure

1 ml of DI water was slowly added to a chromatography column to prevent any trapped air. The slurry solution of Example 3 or 4 was added into the column. 1 ml of resin in the column was measured. Gentle vacuum was applied to remove all but 1-2 cm of solution above the resin. When the resin level fell below the 1 ml mark, more resin slurry was added with additional vacuum. The resin bead was never exposed to air. The packed 1 ml resin was rinsed with approximately 10 ml of DI water to displace the slurry solution and the liquid was allowed to drain until the water level is 1-2 cm above the packed bed. Then the packed I ml resin was flushed with 10 ml of Solution 1. Solution 1 was removed from the column by vacuum and air was pulled through the column for 1 minute. The disposable column containing the wet cake was removed from the manifold.

The wet cake from the column was transferred to an 8oz glass jar with a spatula and 200 mL of solution 2 (2 mg/mL BSA sln.) was added. The sample in the glass jar was gently shaken for 18 h.

After 18 hours, the sample was removed from the shaker and the resin was allowed to settle for 15 minutes.

Determination of the Uptake Binding Capacity:

The BSA binding capacity was determined from the 278 nm UV absorbance of the filtered supernatant BSA solution after 18 hours incubation. A cuvette filled with solution 1 was used to zero the UV spectrometer. The absorbance at 278 nm was measured for all the samples, standards solutions, and control sample (Q Sepharose™ Fast Flow Ion Exchange Resin, GE Healthcare).

Calculation of the BSA Binding Capacity

The values for unbound BSA for both the sample and control were recorded (Q Sepharose FF). The following equation was applied to determine the binding capacity:

Equation 1

$$\text{BSA capacity} = [400 - (\text{sample mg/mL} \times 200)] \times 1 \text{ mL} = \text{mg of BSA/mL of resin}$$

**EXAMPLES**

Example 1: Functionalization of epoxide containing beads with tert-butylhydroperoxide (tBHP). All materials were used as received from the supplier.

15 g of epoxide containing beads (60 $\mu$m polyGMA-GlyDMA beads glycidyl methacrylate (GMA) and glycerol-1,3-dimethacrylate (GlyDMA), produced by a process similar to that disclosed in Example 25 of US patent publication US 2007-0066761 A1) as a wet cake were added to a three neck round bottom flask equipped with a mechanical stirrer followed by the addition of 18 mL of water to form slurry. Tert-butylhydroperoxide (tBHP, Sigma-Aldrich)) and triethylamine (Et$_3$N) (Sigma-Aldrich) were added to the slurry and the reaction mixture was stirred overnight at room temperature under a N$_2$ atmosphere. The beads were filtered off and rinsed with water. The resulting material was stored as slurry in water.

Example 2: Functionalization of epoxide containing beads with peracetic acid

Place 25 g of the epoxide containing beads of Example 1 (GMA/GLYDMA copolymer) wet cake into a 250 ml reaction flask equipped with Teflon stir paddle and set the overhead stirrer to 145 RPM. Add 30g of Milli) Q water, 0.5 g 4 hydroxy tempo, (Aldrich lot 77997KJ), 0.08 g of tributylaime, (Aldrich lot A0256662) and 1.8 g of 37 % peracetic acid into the reactor. Heat the reactor to 50 c over 1 hour and then hold 18 hours 50 c. Cool to room temperature and isolate the beads in a 350 ml fritted glass funnel and rinse with 1 liters of Milli Q water. BSA capacity 162 mg/ml.

Example 3: Functionalization of peroxide modified beads with (3-acrylamidopropyl)-trimethylammonium chloride (AMPTAC).

10 g of peroxide modified beads (obtained from example 1), 13.6 g of AMPTAC (Sigma-Aldrich) and 10 mL of water were added to a three neck round bottom flask equipped with a mechanical stirrer. The solution was purged with N$_2$ for 15 min. The reaction mixture was heated to 85 °C and stirred overnight under N$_2$. The beads were filtered off and rinsed with water. The resulting material was stored as slurry in water.

Example 4: Functionalization of peroxy ester modified beads with (3-acrylamidopropyl)-trimethylammonium chloride (AMPTAC).

Place 25 g of the product of example 2 wet cake into a 250 ml reaction flask equipped with Teflon stir paddle and set the overhead stirrer to 145 RPM. Add 6 g of 75 % (3 acrylamidopropyl) trimethylammonium chloride (Aldrich lot # 02523MH) into the reaction flask with stirring. Then add 0.12 g of ammonium persulfate (Aldrich lot # A0264270) and 13.6 g of Milli Q water into the reactor. Let the mixture stir at room temperature for 1 hour with stirring. Heat the reactor to 80 c over 1 hour, then hold the reactor at 80 c for 5 hours. Cool to 50 c and add 56 mls of 1 NaOH slowly (over 5 mins) to the reactor. Hold for 1 hour at 50 c. Then cool the reactor to room temperature. Isolate the beads in a 350 ml fritted glass funnel and rinse with 3 liters of Milli Q water. BSA capacity 74 mg/ml.

**Claims**

1. A method of preparing an adsorbent material for chromatography comprising:

(i) providing a polymeric substrate comprising a functional group
(ii) covalently attaching a free radical Ligand to the functional group to form a surface reactive group
(iii) reacting by free radical the surface reactive group with a vinyl monomer to form a polymeric ligand.

2. The method of claim 1 wherein the vinyl monomer is selected from the group comprising methacrylates, acrylates, methacrylamides and acrylamides and combinations thereof. The monomers include, but are not limited to, acrylic acid, 2-acrylamido-2-methyl-1-propanesulfonic acid, [3-(methacryloylamino) propyl] trimethylammonium chloride, 2-acrylamido-glycolic acid, itaconic acid or ethyl vinyl ketone, glycidyl methacrylate, N,N -dimethylacrylamide, acrylamide, hydroxypropyl methacrylate, N-phenylacrylamide, hydroxyethyl acrylamide, and combinations thereof

3. The method of claim 1 wherein the polymeric ligand is selected from the group comprising strong cation exchange groups, strong anion exchange groups, weak cation exchange groups, weak anion exchange groups, hydrophobic interaction groups, affinity groups, unfunctional monomers and intermediary monomers capable of further transformation into another functional group, and mixtures thereof.

4. An adsorbent material for chromatography comprising:

a polymeric ligand immobilized onto a polymeric substrate,
wherein the polymeric substrate comprises a functional group covalently attached to a free radical ligand to form a surface reactive group
and further wherein the surface reactive group is reacted by free radical with a vinyl monomer to form a polymeric ligand.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20070066761 A1

### Non-patent literature cited in the description

- **RUIXIN WANG.** Studies on Preparation of PGMA/ Al2O3 and its Effect on Impact Strength of Epoxy Resin. *Journal of Applied Polymer Science,* 2009, vol. 113, 41-48